**Europäisches Patentamt**

**European Patent Office**

**Office européen des brevets**

(11) Veröffentlichungsnummer: **0 257 542 B1**

(12) # EUROPÄISCHE PATENTSCHRIFT

(45) Veröffentlichungstag der Patentschrift: **06.05.92**

(21) Anmeldenummer: **87112023.4**

(22) Anmeldetag: **19.08.87**

(51) Int. Cl.5: **C12N 15/00**, C12N 15/54, C12N 15/64, C12N 9/10, C12P 41/00

(54) **Resistenzgen gegen Phosphinothricin und seine Verwendung.**

(30) Priorität: **23.08.86 DE 3628747**
**03.11.86 DE 3637307**
**16.12.86 DE 3642829**
**08.01.87 DE 3700313**

(43) Veröffentlichungstag der Anmeldung:
**02.03.88 Patentblatt 88/09**

(45) Bekanntmachung des Hinweises auf die Patenterteilung:
**06.05.92 Patentblatt 92/19**

(84) Benannte Vertragsstaaten:
**AT BE CH DE ES FR GB GR IT LI LU NL SE**

(56) Entgegenhaltungen:
**EP-A- 0 242 246**

(73) Patentinhaber: **HOECHST AKTIENGESELL-SCHAFT**
**Postfach 80 03 20**
**W-6230 Frankfurt am Main 80(DE)**

(72) Erfinder: **Strauch, Eckhard**
**Rosenheide 2**
**W-4800 Bielefeld(DE)**
Erfinder: **Wohlleben, Wolfgang, Dr.**
**Menzelstrasse 1**
**W-4800 Bielefeld(DE)**
Erfinder: **Arnold, Walter**
**Am Gottesberg 25**
**W-4800 Bielefeld(DE)**
Erfinder: **Alijah, Renate**
**Kösterkamp 14**
**W-4800 Bielefeld(DE)**
Erfinder: **Pühler, Alfred, Prof. Dr.**
**Am Waldschlösschen 2**
**W-4800 Bielefeld(DE)**
Erfinder: **Wöhner, Gerhard, Dr.**
**Flörsheimer Strasse 27**
**W-6093 Flörsheim am Main(DE)**

EP 0 257 542 B1

UCLA SYMPOSIUM ON MOLECULAR AND CELLULAR BIOLOGY NEW SERIES, Band 62, PLANT GENE SYSTEMS AND THEIR BIOLO-GY, CIBA-GEIGY-UCLA SYMPOSIUM, Tamar-ron, Colorado, 2. - 8. Februar 1987, Seiten 171-181, Alan R. Liss, Inc.; M. VAECK et al.: "Engineering improved crops for agriculture: protection from insects and resistance to herbicides"

GENE, Band 70, 1988, Seiten 25-37, Elsevier Science Publishers B.V. (Biomedical Divi-sion); W. WOHLLEBEN et al.: "Nucleotide se-quence of the phosphinothricin N-acetyltransferase gene from Streptomyces viridochromogenes Tü494 and its expres-sion in Nicotiana tabacum"

Erfinder: **Marquardt, Rüdiger, Dr.**
**Günthersburgallee 69**
**W-6000 Frankfurt am Main(DE)**
Erfinder: **Grabley, Susanne, Dr.**
**Hölderlinstrasse 7**
**W-6240 Königstein/Taunus(DE)**
Erfinder: **Brauer, Dieter, Dr.**
**Berliner Strasse 14**
**W-6093 Flörsheim am Main(DE)**
Erfinder: **Bartsch, Klaus, Dr.**
**Memelstrasse 2**
**W-6233 Kelkheim (Taunus)(DE)**

## Beschreibung

Phosphinothricin (PTC, 2-Amino-4-methylphosphinobuttersäure) ist ein Glutaminsynthetase-Inhibitor. PTC ist ein "Baustein" des Antibiotikums Phosphinothricyl-alanyl-alanin. Dieses Tripeptid (PTT) ist aktiv gegen Gram-positive und Gram-negative Bakterien und auch gegen den Pilz Botrytis cinerea (Bayer et al., Helv. Chim. Acta 55 (1972) 224). PTT wird von dem Stamm Streptomyces viridochromogenes Tü 494 (DSM 40736, DSM 4112) produziert.

Aus der Deutschen Patentschrift 2 717 440 ist es bekannt, daß PTC als Totalherbizid wirkt. In der veröffentlichten PCT-Anmeldung WO 86/02097 sind Pflanzen beschrieben, deren Resistenz gegen PTC darauf zurückzuführen ist, daß sie Glutaminsynthetase überproduzieren. Solche Überproduktionen, beispielsweise infolge einer Genamplifikation, bergen jedoch die Gefahr der Instabilität in sich. Im Falle einer solchen Instabilität ginge also die Überproduktion an Glutaminsynthetase zurück und die kompetitive Inhibitorwirkung des PTC käme wieder zum Zuge.

Die Erfindung, die in den Patentansprüchen definiert ist, bezieht sich demgegenüber auf ein Resistenzgen gegen PTC und seine Verwendung zur Herstellung PTC-resistenter Pflanzen. Darüber hinaus kann dieses Gen auch als Resistenz-Marker Verwendung finden. Weiterhin eignet sich das Gen zur selektiven N-Acetylierung der L-Form von racemischem PTC.

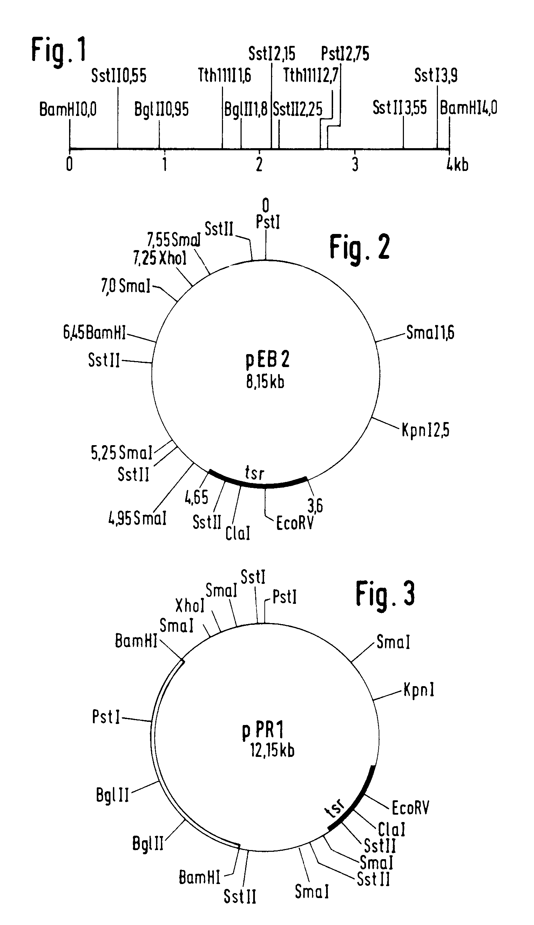

Das erfindungsgemäße Resistenzgen gegen PTC ist erhältlich aus der Gesamt-DNA von auf PTT-Resistenz selektiertem Streptomyces viridochromogenes DSM 4112 durch Schneiden mit BamHI, Klonieren eines 4,0 kb großen Fragments und Selektion auf PTT-Resistenz. Die Restriktionskarte (Figur 1) charakterisiert dieses 4,0 kb-Fragment näher.

Durch Klonieren von Teilbereichen dieses 4 kb-Fragmentes wurde die Lage des Codierbereichs näher eingegrenzt. Hierbei zeigte sich, daß das Resistenzgen auf dem 1,6 kb SstII-SstI-Fragment (Positionen 0,55 bis 2,15 in Fig. 1) liegt. Durch Verdauung mit BglII wird das 0,8 kb große Fragment gewonnen, das nach Einbau in ein Plasmid und Transformation von S. lividans PTT-Resistenz vermittelt. Diese Resistenz ist durch die N-Acetylierung von PTC bedingt.

Die Sequenzierung nach Maxam und Gilbert des 0,8 kb-Fragments ergibt die DNA-Sequenz I (Anhang). Aus den offenen Leserahmen dieser Sequenz läßt sich die Lage des Resistenzgens ermitteln (ab Position 258). Das Ende des Gens liegt beim vorletzten der wiedergegeben Nucleotide (Position 806), d. h. das letzte Nucleotid (Position 807), ist der Beginn des Stop-Codons.

In der DNA-Sequenz I ist die "Shine-Dalgarno-Sequenz" durch Unterstreichen hervorgehoben, ebenso das als Startcodon wirkende GTG. Der maßgebliche Leserahmen ist also in der obersten Zeile ausgedruckt.

Die DNA-Sequenz II zeigt die Restriktionsschnittstellen innerhalb des sequenzierten Gens. Enzyme, die die Sequenz mehr als sechsmal schneiden, sind nicht angegeben.

Das Antibiotikum PTT wird von Bakterien aufgenommen und zu PTC abgebaut. Dieses inhibiert bei Bakterien ebenfalls die Glutaminsynthetase, so daß die Bakterien an Glutaminmangel sterben. PTT-produzierende Bakterien sollten daher einen Mechanismus besitzen, der sie vor der Wirkung des PTT schützt, also entweder die Wiederaufnahme des produzierten PTT verhindert oder eine Modifikation des Abbauproduktes PTC ermöglicht. Überraschenderweise ist der PTT-Produzent S. viridochromogenes DSM 4112 aber gegen sein eigenes Antibiotikum sensitiv. Unerwarteterweise gelang es aber, durch Selektion auf PTT-Resistenz mit der überraschend hohen Rate von etwa $10^{-5}$ Selektanten zu finden, die gegen PTT resistent sind und auch das Untergrundwachstum der benachbarten Kolonien unterdrücken.

Aus der DNA dieser Selektanten wurde eine Genbank angelegt, indem die DNA isoliert, mit BamHI gespalten und in einen Streptomycetenvektor ligiert wurde. Das Ligationsgemisch wurde in den handelsüblichen Stamm S. lividans TK 23 transformiert, wobei je 1 μg Ligationsgemisch etwa 5000 bis 10000 Transformanten mit einem Insert von etwa 1 bis 5 kb erhalten wurden. Unter den Transformanten finden sich PTT-resistente S. lividans-Stämme. Durch Isolierung des Plasmids und Retransformation in S. lividans konnte gezeigt werden, daß die Resistenz plasmidcodiert ist. Das für die Resistenz verantwortliche Gen liegt auf einem 4 kb-BamHI-Fragment (Figur 1). Der Codierbereich ist auf dem 0,8 kb BglII-Fragment lokalisiert. Das BamHI-Fragment enthält keine Schnittstellen für die Enzyme ClaI, EcoRI, EcoRV, HindIII, HpaI, KpnI, PvuI, PvuII und XhoI.

Der Vergleich mit der Restriktionskarte eines nicht näher charakterisierten Resistenzgens aus S. hygroscopicus FERM BP-130/ATCC 21705 (Europäische Patentanmeldung mit der Veröffentlichungsnummer 0 173 327, Figur 7) zeigt, daß das erfindungsgemäße Resistenzgen von dem bekannten Gen verschieden ist, welches auf der Suche nach den PTT-Biosynthesegenen gefunden wurde.

Durch Inkubation von Zellextrakten von S. viridochromogenes DSM 4112 und S. lividans TK 23 einerseits und der PTT-resistenten S. viridochromogenes-Selektante und einer plasmidtragenden S. lividans-Transformante andererseits mit PTC und Acetyl-Coenzym A konnte gezeigt werden, daß die letztgenannten Zellen eine acetylierende Aktivität zeigen. Chromatographische Befunde zeigen, daß die Acetylierung an der Aminogruppe erfolgt.

Da auch in E. coli eine PTT-Resistenz festgestellt werden konnte und der Resistenzmechanismus somit auch in Gramnegativen Bakterien funktioniert, kann eine Resistenz auf Grund von Transportphänomenen ausgeschlossen werden. Das erfindungsgemäße Resistenzgen kann somit nach Kopplung an pflanzliche Promotoren mit geeigneten Vektoren in Pflanzen transformiert und es können so PTC-resistente Pflanzen hergestellt werden.

Die N-Acetylierung von PTC kann auch zur Racemattrennung von synthetischem D,L-PTC genutzt werden, da selektiv nur die L-Form acetyliert wird.

Die Erfindung betrifft somit auch die Verwendung des Resistenzgens zur selektiven N-Acetylierung der L-Form von racemischem PTC.

Die von dem erfindungsgemäßen Resistenzgen kodierte PTCAcetyltransferase kann also dazu benutzt werden, racemisches PTC, wie es beispielsweise nach der deutschen Patentschrift 2 717 440 erhältlich ist, in die optischen Antipoden zu trennen, indem das Racemat der acetylierenden Wirkung dieses Enzyms ausgesetzt wird, wobei selektiv die L-Form angegriffen wird, während die D-Form unverändert bleibt. Das so erhaltene Gemisch kann dann aufgrund seiner unterschiedlichen Eigenschaften in an sich bekannter Weise aufgetrennt werden.

Es ist bekannt, N-Acyl-D,L-Aminosäuren mit gegebenenfalls trägerfixierten Acylasen in Kontakt zu bringen, wobei selektiv die L-Aminosäure freigesetzt wird, die aus dem Gemisch mit der N-Acyl-D-Aminosäure nach Ansäuern mit nicht wassermischbaren Lösemitteln extrahiert werden kann (Britische Patentschrift 1 369 462). Eine entsprechende Auftrennung von N-Acyl-D,L-PTC ist beispielsweise aus der Deutschen Offenlegungsschrift 2 939 269 oder der US-Patentschrift 4 226 941 bekannt.

Das erfindungsgemäß zurückbleibende D-PTC kann in bekannter Weise racemisiert werden (Europäische Patentanmeldung mit der Veröffentlichungsnummer (EP-A) 0 137 371, Beispiel 8), und dann in den Prozeß zurückgeführt werden.

Die Isolierung des Enzyms, worunter hier und im folgenden auch immer der enzymatisch wirksame Teil verstanden werden soll, ist möglich, aber nicht erforderlich. Falls das Enzym isoliert wird, kann es in freier oder trägerfixierter Form eingesetzt werden. Geeignete Träger sind beispielsweise in der EP-A 0 141 223 beschrieben. Zweckmäßig wird jedoch das Enzym nicht isoliert, sondern man setzt beliebige PTC-resistente Zellen ein, die das erfindungsgemäße Enzym exprimieren. So kann zweckmäßig die PTT-resistente Selektante von S. viridochromogenes DSM 4112 eingesetzt werden. Vorteilhaft kann auch eine beliebige, mit dem erfindungsgemäßen Gen transformierte Zelle zum Einsatz gelangen, die in der Lage ist, die PTC-Acetyltransferase zu exprimieren. Das erfindungsgemäße Gen, worunter hier auch aktive Teile desselben verstanden werden, kann hierbei in plasmidintegrierter Form in die Wirtszelle eingebracht werden oder mit anderen üblichen gentechnischen Methoden, beispielsweise durch Transfektion. Zweckmäßig ist beispielsweise der Einbau in ein E. coli-Expressionsplasmid und Transformation von E. coli mit einem solchen Plasmid, beispielsweise nach den aus EP-A 0 163 249 und 0 171 024 bekannten Verfahren.

Zur erfindungsgemäßen N-Acetylierung von L-PTC im Racemat können die Zellen, die die PTC-Acetyltransferase exprimieren, in freier oder fixierter Form eingesetzt werden, wobei die üblichen Fixierungsmethoden Anwendung finden (z.B. Deutsche Offenlegungsschrift 3 237 341 und darin zitierte Literatur).

Die erfindungsgemäße enzymatische Acetylierung von L-PTC erfolgt in der für enzymatische Umsetzungen üblichen Weise, wobei sich die Verfahrensbedingungen an den Gegebenheiten des eingesetzten Organismus orientieren. Grundsätzlich kommen hierfür dieselben Methoden wie für die vorstehend genannten selektiven Entacylierungsverfahren in Betracht.

In den folgenden Beispielen wird die Erfindung näher erläutert. Teile und Prozentangaben beziehen sich auf das Gewicht, sofern keine anderen Angaben gemacht werden.

Beispiel 1: PTT-resistente Selektanten

Der Stamm S. viridochromogenes DSM 4112 wurde auf Minimal-Medium (Hopwood et al., Genetic Manipulation of Streptomyces, A Laboratory Manual, The John Innes Foundation, Norwich, England (1985), S. 233) angezogen und mit PTT in steigenden Konzentrationen versetzt. Bei einer Konzentration von 100 $\mu$g/ml wurde pro etwa $10^5$ Kolonien eine resistente Kolonie gefunden.

Beispiel 2: Herstellung des Vektors

Das Plasmid pSVH1 (Europäische Patentschrift 0 070 522) wird mit BglII geschnitten, das etwa 7,1 kb große Fragment isoliert und mit dem 1,1 kb BclI-Fragment mit der Thiostrepton-Resistenz (Europäische Patentanmeldung mit der Veröffentlichungsnummer 0 158 201) ligiert. Man erhält das 8,15 kb große Plasmid pEB2 (Figur 2).

Beispiel 3: Isolierung des Resistenzgens

Aus den Selektanten gemäß Beispiel 1 isoliert man die Gesamt-DNA und spaltet sie mit BamHI. Das Plasmid pEB2 wird ebenfalls mit BamHI geöffnet, die beiden Ansätze vereinigt und ligiert. Das Ligationsgemisch wird nach S. lividans TK 23 (erhältlich bei der John Innes Foundation) transformiert, wobei je 1 $\mu$g Ligationsgemisch 5000 bis 10000 Transformanten mit einem Insert von etwa 1 - 5 kb erhalten werden. Selektion auf PTT-Resistenz ergibt 2 resistente S. lividans-Kolonien. Aus diesen wird das aufgenommene Plasmid isoliert und mit BamHI geschnitten. Man findet ein 4 kb BamHI-Fragment, welches das für die Resistenz verantwortliche Gen trägt. Dieses Plasmid erhielt die Bezeichnung pPRI (Figur 3).

Durch Retransformation in S. lividans TK 23 kann gezeigt werden, daß die PTT-Resistenz plasmidcodiert ist, da die Transformanten auf Minimalmedium wachsen, das 100 $\mu$g/ml PTT enthält.

Beispiel 4: Nachweis der Inaktivierung von PTC durch N-Acetylierung

Zum Nachweis der acetylierenden Aktivität des klonierten Fragments wurden folgende Stämme untersucht: S. viridochromogenes DSM 40736, S. viridochromogenes (PTT-resistente Mutante), S. lividans TK23 und S. lividans TK 23 (pPR1).

Dazu werden die Stämme in Lysemedium A (Europäische Patentanmeldung mit der Veröffentlichungsnummer 0 158 872, S. 6) angeimpft und 2 Tage bei 30°C im Rundschüttler inkubiert. Nach der Ernte wird 1 mg Mycel in einem geeigneten Puffer (z. B. RS-buffer: C. J. Thompson et al., J. Bacteriol. 151 (1982), 678-685) mit Ultraschall aufgeschlossen. Ein typisches Experiment zur Messung des PTC-Abbaus verläuft folgendermaßen:

250 $\mu$l Rohextrakt werden mit 100 $\mu$l PTC-Lösung (250 $\mu$g/ml) und 50 $\mu$l Acetyl-CoA (4 mg/ml) versetzt und 2 Stunden bei 30°C inkubiert. Die dann noch vorhandenen PTC-Mengen werden durch HPLC gemessen. Dabei ergibt sich folgendes Ergebnis:

| Stamm | nicht umgesetztes PTC / eingesetztes PTC |
|---|---|
| S. lividans TK23 | 100% |
| S. viridochromogenes (DSM 40736) | 72% |
| S. viridochromogenes Selektante | 7% |
| S. lividans TK23 (pPR1) | 31% |

Daß es sich um eine N-Acetylierung des PTC handelt, kann durch Vergleich mit Referenzsubstanzen in der Dünnschichtchromatographie nachgewiesen werden (keine Anfärbung durch Ninhydrin).

DNA-Sequenz I

```
IleTrpSerAspValLeuGlyAlaGlyProValLeuProGlyAspAspPhePheSerLeuGlyGlyThrSerIle
AspLeuGluArgArgProGlyGlyArgSerGlyAlaAlaArgGlyArgLeuLeuLeuProArgArgHisLeuHis
ArgSerGlyAlaThrSerTrpGlyProValArgCysCysProGlyThrThrSerSerProSerAlaAlaProPro
AGATCTGGAGCGACGTCCTGGGGGCCGGTCCGGTGCTGCCCGGGGACGACTTCTTCTCCCTCGGCGGCACCTCCA  75
TCTAGACCTCGCTGCAGGACCCCCGGCCAGGCCACGACGGGCCCCTGCTGAAGAAGAGGGAGCCGCCGTGGAGGT
SerArgSerArgArgGlyProProArgAspProAlaAlaArgProArgSerArgArgGlyArgArgCysArgTrp
   AspProAlaValAspGlnProGlyThrArgHisGlnGlyProValValGluGluGlyGluAlaAlaGlyGlyAsp
   IleGlnLeuSerThrArgProAlaProGlyThrSerGlyProSerSerLysLysGluArgProProValGluMet

   SerAlaLeuArgValValSerArgIleArgLysGluLeuGlyValProLeuArgLeuAlaValIlePheGluThr
   LeuGlyValAlaGlyGlyLeuAlaHisProGlnGlyThrArgArgAlaThrProAlaArgArgAspLeuArgAsp
   SerArgArgCysGlyTrpSerArgAlaSerAlaArgAsnSerAlaCysHisSerGlySerProOP SerSerArg
   TCTCGGCGTTGCGGGTGGTCTCGCGCATCCGCAAGGAACTCGGCGTGCCACTCCGGCTCGCCGTGATCTTCGAGA  150
   AGAGCCGCAACGCCCACCAGAGCGCGTAGGCGTTCCTTGAGCCGCACGGTGAGGCCGAGCGGCACTAGAAGCTCT
   ArgProThrAlaProProArgAlaCysGlyCysProValArgArgAlaValGlyAlaArgArgSerArgArgSer
      ArgArgGlnProHisAspArgAlaAspAlaLeuPheGluAlaHisTrpGluProGluGlyHisAspGluLeuArg
      GluAlaAsnArgThrThrGluArgMetArgLeuSerSerProThrGlySerArgSerAlaThrIleLysSerVal

      ProSerLeuGluAlaValAlaGluSerValLeuArgGluLeuLysGlyThrAM OC ArgGlyAlaArgHisPro
      AlaValProGlySerGlyGlyArgIleArgThrProArgThrGluGlyAspValValLysArgCysProProPro
   ArgArgProTrpLysArgTrpProAsnProTyrSerAlaAsnOP ArgGlyArgSerLysGluValProAlaThr
   CGCCGTCCCTGGAAGCGGTGGCCGAATCCGTACTCCGCGAACTGAAGGGGACGTAGTAAAGAGGTGCCCGCCACC  225
   GCGGCAGGGACCTTCGCCACCGGCTTAGGCATGAGGCGCTTGACTTCCCCTGCATCATTTCTCCACGGGCGGTGG
   AlaThrGlyProLeuProProArgIleArgValGlyArgValSerProSerThrThrPheLeuHisGlyGlyGly
      ArgGlyGlnPheArgHisGlyPheGlyTyrGluAlaPheGlnLeuProArgLeuLeuSerThrGlyAlaValArg
      GlyAspArgSerAlaThrAlaSerAspThrSerArgSerSerPheProValTyrTyrLeuProAlaArgTrpGly

      LeuSerGlnAsnThrGluGlyArgProHisValSerProGluArgArgProValGluIleArgProAlaThrAla
      AlaPheAlaGluHisArgArgLysThrThrArgGluProArgThrThrProGlyArgAspProSerArgHisArg
   ArgPheArgArgThrProLysGluAspHisThrOP AlaGlnAsnAspAlaArgSerArgSerValProProPro
   CGCTTTCGCAGAACACCGAAGGAAGACCACACGTGAGCCCAGAACGACGCCCGGTCGAGATCCGTCCCGCCACCG  300
   GCGAAAGCGTCTTGTGGCTTCCTTCTGGTGTGCACTCGGGTCTTGCTGCGGGCCAGCTCTAGGCAGGGCGGTGGC
   AlaLysAlaSerCysArgLeuPheValValArgSerGlyLeuValValGlyProArgSerGlyAspArgTrpArg
      LysArgLeuValGlyPheSerSerTrpValHisAlaTrpPheSerAlaArgAspLeuAspThrGlyGlyGlyGly
      SerGluCysPheValSerProLeuGlyCysThrLeuGlySerArgArgGlyThrSerIleArgGlyAlaValAla

      AlaAspMetAlaAlaValCysAspIleValAsnHisTyrIleGluThrSerThrValAsnPheArgThrGluPro
      ArgArgHisGlyGlyGlyLeuArgHisArgGlnSerLeuHisArgAspGluHisGlyGlnLeuProTyrGlyAla
   ProProThrTrpArgArgSerAlaThrSerSerIleThrThrSerArgArgAlaArgSerThrSerValArgSer
   CCGCCGACATGGCGGCGGTCTGCGACATCGTCAATCACTACATCGAGACGAGCACGGTCAACTTCCGTACGGAGC  375
   GGCGGCTGTACCGCCGCCAGACGCTGTAGCAGTTAGTGATGTAGCTCTGCTCGTGCCAGTTGAAGGCATGCCTCG
   ArgArgCysProProProArgArgCysArgOP AspSerCysArgSerSerCysProOP SerGlyTyrProAla
      GlyValHisArgArgAspAlaValAspAspIleValValAspLeuArgAlaArgAspValGluThrArgLeuArg
      AlaSerMetAlaAlaThrGlnSerMetThrLeuOP AM MetSerValLeuValThrLeuLysArgValSerGly

      GlnThrProGlnGluTrpIleAspAspLeuGluArgLeuGlnAspArgTyrProTrpLeuValAlaGluValGlu
      AlaAspSerAlaGlyValAspArgArgProGlyAlaProProGlyProLeuProLeuAlaArgArgArgGlyGly
   ArgArgLeuArgArgSerGlySerThrThrTrpSerAlaSerArgThrAlaThrProGlySerSerProArgTrp
   CGCAGACTCCGCAGGAGTGGATCGACGACCTGGAGCGCCTCCAGGACCGCTACCCCTGGCTCGTCGCCGAGGTGG  450
   GCGTCTGAGGCGTCCTCACCTAGCTGCTGGACCTCGCGGAGGTCCTGGCGATGGGGACCGAGCAGCGGCTCCACC
   AlaSerGluAlaProThrSerArgArgGlyProAlaGlyGlyProGlySerGlyArgAlaArgArgArgProPro
      LeuSerArgLeuLeuProAspValValGlnLeuAlaGluLeuValAlaValGlyProGluAspGlyLeuHisLeu
      CysValGlyCysSerHisIleSerSerArgSerArgArgTrpSerArgAM GlyGlnSerThrAlaSerThrSer
```

DNA-Sequenz I    (Fortsetzung)


GlyValValAlaGlyIleAlaTyrAlaGlyProTrpLysAlaArgAsnAlaTyrAspTrpThrValGluSerThr
GlyArgArgArgArgHisArgLeuArgArgProLeuGluGlyProGlnArgLeuArgLeuAspArgArgValAsp
ArgAlaSerSerProAlaSerProThrProAlaProGlyArgProAlaThrProThrThrGlyProSerSerArg
AGGGCGTCGTCGCCGGCATCGCCTACGCCGGCCCCTGGAAGGCCCGCAACGCCTACGACTGGACCGTCGAGTCGA 525
TCCCGCAGCAGCGGCCGTAGCGGATGCGGCCGGGGACCTTCCGGGCGTTGCGGATGCTGACCTGGCAGCTCAGCT
ProArgArgArgArgCysArgArgArgArgGlyArgSerProGlyCysArgArgArgSerSerArgArgThrSer
AlaAspAspGlyAlaAspGlyValGlyAlaGlyProLeuGlyAlaValGlyValValProGlyAspLeuArgArg
ProThrThrAlaProMetAlaAM AlaProGlyGlnPheAlaArgLeuAlaAM SerGlnValThrSerAspVal


ValTyrValSerHisArgHisGlnArgLeuGlyLeuGlySerThrLeuTyrThrHisLeuLeuLysSerMetGlu
GlyValArgLeuProProAlaProAlaAlaArgThrGlyLeuHisProLeuHisProProAlaGluValHisGly
ArgCysThrSerProThrGlyThrSerGlySerAspTrpAlaProProSerThrProThrCysOP SerProTrp
CGGTGTACGTCTCCCACCGGCACCAGCGGCTCGGACTGGGCTCCACCCTCTACACCCACCTGCTGAAGTCCATGG 600
GCCACATGCAGAGGGTGGCCGTGGTCGCCGAGCCTGACCCGAGGTGGGAGATGTGGGTGGACGACTTCAGGTACC
ProThrArgArgGlyGlyAlaGlyAlaAlaArgValProSerTrpGlyArgCysGlyGlyAlaSerThrTrpPro
HisValAspGlyValProValLeuProGluSerGlnAlaGlyGlyGluValGlyValGlnGlnLeuGlyHisLeu
ThrTyrThrGluTrpArgCysTrpArgSerProSerProGluValArgAM ValTrpArgSerPheAspMetSer


AlaGlnGlyPheLysSerValValAlaValIleGlyLeuProAsnAspProSerValArgLeuHisGluAlaLeu
GlyProGlyLeuGlnGluArgGlyArgArgHisArgThrAlaGlnArgProGluArgAlaProAlaArgGlyAla
ArgProArgAlaSerArgAlaTrpSerProSerSerAspCysProThrThrArgAlaCysAlaCysThrArgArg
AGGCCCAGGGCTTCAAGAGCGTGGTCGCCGTCATCGGACTGCCCAACGACCCGAGCGTGCGCCTGCACGAGGCGC 675
TCCGGGTCCCGAAGTTCTCGCACCAGCGGCAGTAGCCTGACGGGTTGCTGGGCTCGCACGCGGACGTGCTCCGCG
ProGlyProSerOP SerArgProArgArgOP ArgValAlaTrpArgGlySerArgAlaGlyAlaArgProAla
GlyLeuAlaGluLeuAlaHisAspGlyAspAspSerGlnGlyValValArgAlaHisAlaGlnValLeuArgGlu
AlaTrpProLysLeuLeuThrThrAlaThrMetProSerGlyLeuSerGlyLeuThrArgArgCysSerAlaSer


GlyTyrThrAlaArgGlyThrLeuArgAlaAlaGlyTyrLysHisGlyGlyTrpHisAspValGlyPheTrpGln
ArgIleHisArgAlaArgAspAlaAlaGlySerArgLeuGlnAlaArgGlyLeuAlaArgArgGlyValLeuAla
SerAspThrProArgAlaGlyArgCysGlyGlnProAlaThrSerThrGlyAlaGlyThrThrTrpGlySerGly
TCGGATACACCGCGCGCGGGACGCTGCGGGCAGCCGGCTACAAGCACGGGGGCTGGCACGACGTGGGGTTCTGGC 750
AGCCTATGTGGCGCGCGCCCTGCGACGCCCGTCGGCCGATGTTCGTGCCCCCGACCGTGCTGCACCCCAAGACCG
ArgIleCysArgAlaArgSerAlaAlaProLeuArgSerCysAlaArgProSerAlaArgArgProThrArgAla
SerValGlyArgAlaProArgGlnProCysGlyAlaValLeuValProAlaProValValHisProGluProLeu
ProTyrValAlaArgProValSerArgAlaAlaProAM LeuCysProProGlnCysSerThrProAsnGlnCys


ArgAspPheGluLeuProAlaProProArgProValArgProValThrGlnIle
AlaArgLeuArgAlaAlaGlyProAlaProProArgProAlaArgHisThrAsp
SerAlaThrSerSerCysArgProArgProAlaProSerGlyProSerHisArgSer
AGCGCGACTTCGAGCTGCCGGCCCCGCCCCGCCCCGTCCGGCCCGTCACACAGATCT 807
TCGCGCTGAAGCTCGACGGCCGGGGCGGGGCGGGGCAGGCCGGGCAGTGTGTCTAGA
AlaArgSerArgAlaAlaProGlyAlaGlyGlyArgGlyAlaArgOP ValSerArg
AlaValGluLeuGlnArgGlyArgGlyAlaGlyAspProGlyAspCysLeuAsp
ArgSerLysSerSerGlyAlaGlyGlyArgGlyThrArgGlyThrValCysIle

DNA-Sequenz II

AGATCTGGAGCGACGTCCTGGGGGCCGGTCCGGTGCTGCCCGGGGACGACTTCTTCTCCC
TCTAGACCTCGCTGCAGGACCCCCGGCCAGGCCACGACGGGCCCCTGCTGAAGAAGAGGG
^^ ^^ ^ ^^ ^ ^ ^^ ^ ^

1 BGLII XHOII, 2 DPNI SAU3A, 5 GSUI, 12 AATII ACYI, 13 MAEII
, 17 APYI ECORII, 26 RSRII, 27 AVAII, 35 BBVI, 39 AVAI NCII
SMAI, 40 NCII, 52 MBOII, 59 MNLI,

TCGGCGGCACCTCCATCTCGGCGTTGCGGGTGGTCTCGCGCATCCGCAAGGAACTCGGCG
AGCCGCCGTGGAGGTAGAGCCGCAACGCCCACCAGAGCGCGTAGGCGTTCCTTGAGCCGC
^ ^ ^ ^^

66 HGICI, 70 MNLI, 97 FNUDII, 100 SFANI, 101 FOKI,

TGCCACTCCGGCTCGCCGTGATCTTCGAGACGCCGTCCCTGGAAGCGGTGGCCGAATCCG
ACGGTGAGGCCGAGCGGCACTAGAAGCTCTGCGGCAGGGACCTTCGCCACCGGCTTAGGC
^ ^ ^ ^ ^ ^ ^ ^

122 BGLI, 140 DPNI SAU3A, 142 MBOII, 149 ACYI HGAI TTH111I,
158 APYI ECORII, 169 CFRI GDIII, 174 HINFI, 180 RSAI,

TACTCCGCGAACTGAAGGGGACGTAGTAAAGAGGTGCCCGCCACCCGCTTTCGCAGAACA
ATGAGGCGCTTGACTTCCCCTGCATCATTTCTCCACGGGCGGTGGGCGAAAGCGTCTTGT
^ ^ ^ ^^

186 FNUDII, 201 MAEII, 211 MNLI, 213 HGICI, 214 SDUI,

CCGAAGGAAGACCACACGTGAGCCCAGAACGACGCCCGGTCGAGATCCGTCCCGCCACCG
GGCTTCCTTCTGGTGTGCACTCGGGTCTTGCTGCGGGCCAGCTCTAGGCAGGGCGGTGGC
^ ^^^ ^ ^ ^^

247 MBOII, 254 AFLIII, 255 PMACI, 256 MAEII, 260 HGIJII SDUI
, 271 ACYI HGAI, 275 NCII, 283 XHOII, 284 BINI DPNI SAU3A,

CCGCCGACATGGCGGCGGTCTGCGACATCGTCAATCACTACATCGAGACGAGCACGGTCA
GGCGGCTGTACCGCCGCCAGACGCTGTAGCAGTTAGTGATGTAGCTCTGCTCGTGCCAGT
^ . ^ ^ ^ ^

303 BGLI, 308 NLAIII, 324 TTH111I, 350 HGIAI SDUI, 357 HINCI
I,

ACTTCCGTACGGAGCCGCAGACTCCGCAGGAGTGGATCGACGACCTGGAGCGCCTCCAGG
TGAAGGCATGCCTCGGCGTCTGAGGCGTCCTCACCTAGCTGCTGGACCTCGCGGAGGTCC
^ ^ ^^ ^^ ^ ^^ ^ ^

367 RSAI, 380 HINFI, 394 BINI, 395 DPNI SAU3A, 404 APYI ECOR
II, 405 GSUI, 409 HAEII, 413 MNLI, 414 GSUI, 416 APYI ECORII
, 419 AVAII,

ACCGCTACCCCTGGCTCGTCGCCGAGGTGGAGGGCGTCGTCGCCGGCATCGCCTACGCCG
TGGCGATGGGGACCGAGCAGCGGCTCCACCTCCCGCAGCAGCGGCCGTAGCGGATGCGGC
^ ^ ^^ ^ ^

430 APYI ECORII, 444 MNLI, 450 MNLI, 453 ACYI, 454 HGAI, 462
NAEI, 466 SFANI, 477 NAEI,

GCCCCTGGAAGGCCCGCAACGCCTACGACTGGACCGTCGAGTCGACGGTGTACGTCTCCC
CGGGGACCTTCCGGGCGTTGCGGATGCTGACCTGGCAGCTCAGCTGCCACATGCAGAGGG
^ ^ ^ ^ ^^ ^

484 APYI ECORII, 511 AVAII, 519 HINFI, 521 ACCI HINCII SALI,
530 RSAI, 532 MAEII,

DNA-Sequenz II    (Fortsetzung)

```
541  ACCGGCACCAGCGGCTCGGACTGGGCTCCACCCTCTACACCCACCTGCTGAAGTCCᵗ
     TGGCCGTGGTCGCCGAGCCTGACCCGAGGTGGGAGATGTGGGTGGACGACTTCAGGTᵗ
         ^     ^              ^        ^       ^     ^            ^ ^
     544 HGICI, 549 NSPBII, 563 HGIJII SDUI, 572 MNLI, 578 TAQII,
      583 BSPMI, 595 NCOI STYI, 596 NLAIII, 600 MNLI,

601  AGGCCCAGGGCTTCAAGAGCGTGGTCGCCGTCATCGGACTGCCCAACGACCCGAGCGTGC
     TCCGGGTCCCGAAGTTCTCGCACCAGCGGCAGTAGCCTGACGGGTTGCTGGGCTCGCACG
         ^                                                     ^
     605 APYI ECORII, 650 AVAI,

661  GCCTGCACGAGGCGCTCGGATACACCGCGCGCGGGACGCTGCGGGCAGCCGGCTACAAGC
     CGGACGTGCTCCGCGAGCCTATGTGGCGCGCGCCCTGCGACGCCCGTCGGCCGATGTTCG
         ^   ^              ^^^ ^        ^           ^   ^          ^
     669 MNLI, 671 HAEII, 686 FNUDII, 687 BSSHII, 688 FNUDII, 690
      FNUDII, 695 HGAI, 698 BBVI, 705 BBVI, 708 NAEI, 716 TTHI11I
     I,

721  ACGGGGGCTGGCACGACGTGGGGTTCTGGCAGCGCGACTTCGAGCTGCCGGCCCCGCCCC
     TGCCCCCGACCGTGCTGCACCCCAAGACCGTCGCGCTGAAGCTCGACGGCCGGGGCGGGG
         ^     ^              ^     ^         ^^  ^
     732 DRAIII, 736 MAEII, 749 BBVI, 753 FNUDII, 763 ALUI, 764 B
     BVI, 767 NAEI,

781  GCCCCGTCCGGCCCGTCACACAGATCT
     CGGGGCAGGCCGGGCAGTGTGTCTAGA
         ^            ^ ^
     795 MAEIII, 802 BGLII XHOII, 803 DPNI SAU3A,
```

**Patentansprüche**

**Patentansprüche für folgende Vertragsstaaten : BE, CH, DE, FR, GB, GR, IT, LI, LU, NL, SE**

1.  Resistenzgen gegen Phosphinothricin (PTC), erhältlich aus der Gesamt-DNA von auf Phosphinothricyl-alanyl-alanin (PTT)-Resistenz selektiertem Streptomyces viridochromogenes DSM 4112 durch Schneiden mit BamHI, Klonieren eines 4,0 kb großen Fragments und Selektion auf PTT-Resistenz.

2.  Gen nach Anspruch 1, gekennzeichnet durch die Restriktionskarte gemäß Figur 1.

3.  Gen nach Anspruch 1 und 2, gekennzeichnet durch die DNA-Sequenz I (Anhang), Position 258-806.

4.  Verwendung des Gens nach Anspruch 1 bis 3 zur Herstellung PTC-resistenter Pflanzen.

5.  Verwendung des Gens nach Anspruch 1 bis 3 als PTT-Resistenz-Marker in Bakterien.

6.  Verwendung des Gens nach Anspruch 1 bis 3 als PTC-Resistenzmarker in Pflanzenzellen.

7.  Verwendung des Gens nach Anspruch 1 bis 3 zur selektiven N-Acetylierung der L-Form von racemischem PTC.

8.  PTC-resistente Pflanzen, gekennzeichnet durch ein Gen nach einem der Ansprüche 1 bis 3.

**Patentansprüche für folgende Vertragsstaaten : AT, ES**

1. Verfahren zur Gewinnung eines Resistenzgens gegen Phosphinothricin (PTC), dadurch gekennzeichnet, daß man aus der Gesamt-DNA von auf Phosphinothricyl-alanyl-alanin (PTT)-Resistenz selektiertem Streptomyces viridochromogenes DSM 4112 durch Schneiden mit BamHI ein 4,0 kb großes Fragment isoliert, dieses ganz oder ein das Resistenzgen enthaltendes Teilfragment davon kloniert und auf PTT-Resistenz selektiert.

2. Verfahren nach Anspruch 1, dadurch gekennzeichnet, daß man das innerhalb des 4,0 kb großen BamHI-Fragments gelegene, 0,8 kb große BglII-Fragment kloniert.

3. Verwendung des nach Anspruch 1 oder 2 erhältlichen Gens zur Herstellung PTC-resistenter Pflanzen.

4. Verwendung des nach Anspruch 1 oder 2 erhältlichen Gens als PTT-Resistenzmarker in Bakterien.

5. Verwendung des nach Anspruch 1 oder 2 erhältlichen Gens als PTC-Resistenzmarker in Pflanzenzellen.

6. Verwendung des nach Anspruch 1 oder 2 erhältlichen Gens zur selektiven N-Acetylierung der L-Form von racemischem PTC.

**Claims**

**Claims for the following Contracting States : BE, CH, DE, FR, GB, GR, IT, LI, LU, NL, SE**

1. A phosphinothricin (PTC) resistance gene which can be obtained by cutting, with BamHI, the total DNA from Streptomyces viridochromogenes DSM 4112 which has been selected for phosphinothricyl-alanyl-alanine (PTT) resistance, by cloning a fragment 4.0 kb in size, and by selection for PTT resistance.

2. The gene as claimed in claim 1, which has the restriction map shown in Figure 1.

3. The gene as claimed in claim 1 and 2, which has the DNA sequence I (annex), position 258-806.

4. The use of the gene as claimed in claims 1 to 3 for the production of PTC-resistant plants.

5. The use of the gene as claimed in claims 1 to 3 as PTT resistance marker in bacteria.

6. The use of the gene as claimed in claims 1 to 3 as PTC resistance marker in plant cells.

7. The use of the gene as claimed in claims 1 to 3 for the selective N-acetylation of the L-form of racemic PTC.

8. PTC-resistant plants which have a gene as claimed in any of claims 1 to 3.

**Claims for the following Contracting States : AT, ES**

1. A process for obtaining a phosphinothricin (PTC) resistance gene which comprises isolation of a fragment 4.0 kb in size by cutting, with BamHI, from the total DNA from Streptomyces viridochromogenes DSM 4112 which has been selected for phosphinothricyl-alanyl-alanine (PTT) resistance, cloning the whole of this fragment or a part thereof containing the resistance gene, and selecting for PTT resistance.

2. The process as claimed in claim 1, wherein the BglII fragment 0.8 kb in size cut within the BamHI fragment 4.0 kb in size is cloned.

3. The use of the gene obtainable as claimed in claim 1 or 2 for the production of PTC-resistant plants.

4. The use of the gene obtainable as claimed in claim 1 or 2 as PTT resistance marker in bacteria.

EP 0 257 542 B1

5. The use of the gene obtainable as claimed in claim 1 or 2 as PTC resistance marker in plant cells.

6. The use of the gene obtainable as claimed in claim 1 or 2 for selective N-acetylation of the L-form of racemic PTC.

**Revendications**

**Revendications pour les Etats contractants suivants : BE, CH, DE, FR, GB, GR, IT, LI, LU, NL, SE**

1. Gène de résistance à la phosphinothricine (PTC), obtenu, à partir de l'ADN total de Streptomyces viridochromogenes DSM 4112 sélectionné pour sa résistance à la phosphinothricyl-alanyl-alanine (PTT), par coupure avec BamHI, clonage d'un fragment de taille 4,0 kb et sélection pour la résistance au PTT.

2. Gène selon la revendication 1, caractérisé par la carte de restriction de la Figure 1.

3. Gène selon la revendication 1 ou 2, caractérisé par la séquence d'ADN I (annexe), position 258-806.

4. Utilisation du gène selon une des revendications 1 à 3, pour produire des plantes résistantes à la PTC.

5. Utilisation du gène selon une des revendications 1 à 3, comme marqueur de la résistance au PTT chez les bactéries.

6. Utilisation du gène selon une des revendications 1 à 3, comme marqueur de la résistance au PTT dans les cellules végétales.

7. Utilisation du gène selon une des revendications 1 à 3, pour la N-acétylation sélective de la forme L de la PTC racémique.

8. Plantes résistantes à la PTC, caractérisées par un gène selon une des revendications 1 à 3.

**Revendications pour les Etats contractants suivants : AT, ES**

1. Procédé d'obtention d'un gène de résistance à la phosphinothricine (PTC), caractérisé en ce que, en partant de l'ADN total de Streptomyces viridochromogenes DSM 4112 sélectionné pour sa résistance à la phosphinothricyl-alanyl-alanine (PTT), on isole, par coupure avec BamHI, un fragment de taille 4,0 kb, on clone l'intégralité de ce dernier ou seulement un de ses segments contenant le gène de résistance, et on le sélectionne pour sa résistance au PTT.

2. Procédé selon la revendication 1, caractérisé en ce qu'on clone le fragment BglII de taille 0,8 kb, présent à l'intérieur du fragment BamHI de taille 4,0 kb.

3. Utilisation du gène obtenu selon une des revendications 1 ou 2, pour produire des plantes résistantes à la PTC.

4. Utilisation du gène obtenu selon une des revendications 1 ou 2, comme marqueur de la résistance au PTT chez les bactéries.

5. Utilisation du gène obtenu selon une des revendications 1 ou 2, comme marqueur de la résistance au PTC dans les cellules végétales.

6. Utilisation du gène obtenu selon une des revendications 1 ou 2, pour la N-acétylation sélective de la forme L de la PTC racémique.

11

# Fig.1

# Fig. 2

pEB 2
8,15kb

# Fig. 3

p PR1
12,15kb